# EUROPÄISCHE PATENTSCHRIFT

(11) **EP 1 628 752 B1**
(45) Veröffentlichungstag und Bekanntmachung des Hinweises auf die Patenterteilung: **29.12.2010**
(21) Anmeldenummer: 04731904.1
(22) Anmeldetag: 10.05.2004
(51) Int. Cl.: B01J 19/00, C07B 41/00, C07D 301/19, B01J 29/89

(54) **VERFAHREN ZUR UMSETZUNG EINER ORGANISCHEN VERBINDUNG MIT EINEM HYDROPEROXID**
METHOD FOR REACTING AN ORGANIC COMPOUND WITH A HYDROPEROXIDE
PROCEDE POUR FAIRE REAGIR UN COMPOSE ORGANIQUE AVEC UN HYDROPEROXYDE

(30) Priorität: 08.05.2003 DE 10320634
(43) Veröffentlichungstag der Anmeldung: 01.03.2006
(73) Patentinhaber: BASF SE, 67056 Ludwigshafen (DE)
(72) Erfinder: MÜLLER, Ulrich, 67435 Neustadt (DE); BASSLER, Peter, 68519 Viernheim (DE); TELES, Joaquim H., 67166 Otterstadt (DE); RIEBER, Nobert, 68259 Mannheim (DE)
(86) Internationale Anmeldenummer: PCT/EP2004/004973
(87) Internationale Veröffentlichungsnummer: WO 2004/098769

(56) Entgegenhaltungen:
- WO-A-01/10855
- WO-A-02/08214
- WO-A-94/02245
- US-A- 4 547 602
- US-A- 4 861 932

## Beschreibung

Die vorliegende Erfindung betrifft ein Verfahren zur Umsetzung einer organischen Verbindung mit einem Hydroperoxid, das sich dadurch auszeichnet, dass mindestens zwei voneinander verschiedene Titanzeolith-Katalysatoren eingesetzt werden. Mindestens zwei der voneinander verschiedenen Katalysatoren sind hierbei räumlich voneinander getrennt, wobei die räumliche Trennung beispielsweise dadurch erzielt werden kann, dass sich die voneinander verschiedenen Katalysatoren in unterschiedlichen Reaktoren befinden, die untereinander seriell verschaltet sind, oder sich in einem einzigen Reaktor befinden und in diesem räumlich voneinander getrennt sind.

Die Umsetzung von organischen Verbindungen mit Hydroperoxiden ist im Stand der Technik in zahlreichen Dokumenten behandelt. Im Allgemeinen werden dort jedoch ausschließlich solche Verfahrensführungen diskutiert, bei denen zur Umsetzung der organischen Verbindung mit dem Hydroperoxid ein einziger Katalysator verwendet wird.

Die WO 00/07965 der Anmelderin betrifft beispielsweise ein Verfahren zur Umsetzung einer organischen Verbindung mit einem Hydroperoxid, wobei unter anderem kursorisch erwähnt wird, dass zur größeren Effizienz dieser Umsetzung ein oder mehrere geeignete Katalysatoren zugesetzt werden können. Darüber hinaus finden sich in der WO 00/07965 jedoch keine Angaben darüber, wie ein Verfahren, in dem mehr als ein Katalysator eingesetzt wird, ausgestaltet sein könnte. Demgemäß betrifft dieses Dokument auch in den explizit beschriebenen Ausführungsformen ein Verfahren, bei dem die organische Verbindung mit einem heterogenen Katalysator in Kontakt gebracht wird.

Die WO 01/72729 der Anmelderin betrifft ebenfalls ein Verfahren zur Umsetzung einer organischen Verbindung mit einem Hydroperoxid, das auf eine möglichst effiziente Katalysatorregenerierung abzielt. In diesem Zusammenhang sind unter anderem Ausführungsformen beschrieben, bei denen neben der zwingenden Anwesenheit von mindestens zwei parallelen Reaktoren mindestens zwei Reaktoren in Serie hintereinander geschaltet sind. In unterschiedlichen parallelen Reaktoren können sich gemäß diesem Verfahren Katalysatoren der gleichen Art, aber unterschiedlicher Reaktivität befinden. Über voneinander verschiedene Katalysatoren enthält die WO 01/72729 keine Angaben.

Die WO 01/10855 der Anmelderin beschäftigt sich ebenfalls mit einem Verfahren zur Umsetzung einer organischen Verbindung mit einem Hydroperoxid, in dem inhomogene Reaktionsbedingungen generiert werden. In dem dort beschriebenen Verfahren werden im Reaktionsmedium sowohl der pH-Wert als auch die Temperatur und gegebenenfalls auch der Druck verändert. Über eine Variation des im Verfahren eingesetzten Katalysators findet sich in der WO 01/10855 kein Hinweis.

Die US 4,861,932 betrifft ein Verfahren zur Umwandlung von Paraffinen in Aromaten, wobei zwei Umwandlungszonen mit unterschiedlichen Zeolith-Katalysatoren verwendet werden.

Die US 4,547,602 betrifft ein integriertes Verfahren zur Umwandlung von Oxygenaten und insbesondere von Methanol in schwere Kohlenwasserstoffe, wobei ein zweistufiges Verfahren der katalytischen Umwandlung in unterschiedlichen Reaktionszonen Verwendung findet.

Demgemäß betrifft die vorliegende Erfindung ein Verfahren zur katalysierten Umsetzung einer organischen Verbindung mit einem Hydroperoxid in mindestens einem Reaktor unter Verwendung von mindestens zwei voneinander verschiedenen Titanzeolith Katalysatoren, dadurch gekennzeichnet, dass mindestens zwei der voneinander verschiedenen Zeolith-Katalysatoren räumlich voneinander getrennt eingesetzt werden.

Im Rahmen des erfindungsgemäßen Verfahrens können als Titanzeolith Katalysatoren zum einen das zeolithische Material selbst, zum anderen aus dem zeolithischen Material hergestellte Formkörper oder auch Mischungen aus zeolithischem Material an sich und aus zeolithischem Material hergestellten Formkörpern verwendet werden. Der Begriff "voneinander verschiedene Zeolith-Katalysatoren", wie er im Rahmen der vorliegenden Erfindung verwendet wird, kann sich daher einerseits auf Unterschiede zwischen zeolithischen Materialien und andererseits auf Unterschiede hinsichtlich der Formkörpereigenschaften beziehen.

Unterschiede hinsichtlich der zeolithischen Materialien sind beispielsweise
- der Titangehalt des zeolithischen Materials;
- der Gehalt an anderen chemischen Elementen neben Titan;
- die Porosität des Zeolithen, wobei sich hinsichtlich der Porosität beispielsweise die Geometrien der Poren der voneinander verschiedenen Zeolith-Katalysatoren unterscheiden und diese demgemäß beispielsweise unterschiedliche Porenvolumina, unterschiedliche Porendurchmesser oder unterschiedliche Oberflächen der Poren aufweisen; ebenso können sich die Zeolithen in der Porenverteilung unterscheiden.
- die Kristallstruktur des zeolithischen Materials;
- die Oberflächenmodifizierung des zeolithischen Materials;
- die Acidität des zeolithischen Materials. Unterschiede hinsichtlich der Formkörper sind beispielsweise
- die Geometrie der Katalysatorformkörper;
- die Porosität der Formkörper, wobei sich hinsichtlich der Porosität beispielsweise die Geometrien der Poren der voneinander verschiedenen Formkörper unterscheiden und diese demgemäß beispielsweise unterschiedliche Porenvolumina, unterschiedliche Porendurchmesser oder unterschiedliche Oberflächen der Poren aufweisen; ebenso können sich die Formkörper in der Porenverteilung unterscheiden.
- die mechanische Festigkeit der Formkörper;
- der Bindergehalt der Katalysatorformkörper;
- der Typ des zur Herstellung der Katalysatorformkörper verwendeten Bindermaterials;
- der Gehalt der Katalysatorformkörper an katalytisch aktivem, zeolithischem Material;
- der Kohlenstoffgehalt der Formkörper.

Der Begriff "voneinander verschiedene Zeolith-Katalysatoren" umfasst im Rahmen der vorliegenden Erfindung auch zwei voneinander verschiedene Katalysatormischungen, wobei eine Mischung mindestens zwei unterschiedliche Formkörper oder mindestens zwei unterschiedliche zeolithische Materialien als solche oder mindestens einen Formkörper und mindestens ein zeolithisches Material als solches enthalten kann. Voneinander verschiedene Katalysatormischungen bezeichnen somit oben beschriebene Mischungen, die sich
- entweder in mindestens einem der oben beispielhaft beschriebenen Unterscheidungsmerkmale bezüglich des zeolithischen Materials oder der Formkörper
- oder im Mischungsverhältnis der in der Mischung enthaltenen Komponenten
- oder sowohl in mindestens einem der oben beispielhaft beschrieben Unterscheidungsmerkmale bezüglich des zeolithischen Materials oder der Formkörper als auch im Mischungsverhältnis der in der Mischung enthaltenen Komponenten
unterscheiden.

Der Begriff "räumlich voneinander getrennt", wie er im Rahmen der vorliegenden Erfindung verwendet wird, bezeichnet Ausführungsformen, bei denen die Vorrichtung, in der die Umsetzung durchgeführt wird, mindestens zwei Kompartimente aufweist, wobei eines der Kompartimente einen Titanzeolith-Katalysator enthält und mindestens ein anderes Kompartiment mindestens einen weiteren Titanzeolith-Katalysator enthält, der sich von dem im ersten Kompartiment enthaltenen Zeolith-Katalysatoren unterscheidet.

Eine solche Kompartimentierung kann beispielsweise in einem einzigen Reaktor realisiert werden, wobei wiederum verschiedene Ausführungsformen der Kompartimentierung möglich sind.

So kann die Kompartimentierung in dem einzigen Reaktor beispielsweise derart erreicht werden, dass zwei oder mehr unterschiedliche Zonen des Reaktors mit jeweils unterschiedlichen Titanzeolith-Katalysatoren ausgestattet werden. Dabei können die unterschiedlichen Zonen des Reaktors zur räumlichen Trennung der unterschiedlichen Zeolith-Katalysatoren durch jeweils mindestens eine mechanische Trennvorrichtung voneinander getrennt sein. Zwischen zwei Zonen können demgemäß eine oder mehr gleiche oder voneinander verschiedene mechanische Trennvorrichtungen vorgesehen werden. Bei drei oder mehr Zonen können zur Trennung der verschiedenen Zonen jeweils die gleichen oder jeweils verschiedene Trennvorrichtungen vorgesehen werden.

Als mechanische Trennvorrichtungen sind beispielsweise Siebböden wie beispielsweise Siebbödenbleche oder Siebbödennetze oder beispielsweise in Destillationskolonnen verwendete Packungen oder Füllkörpergestricke zu nennen, die gemäß einer besonders bevorzugten Ausführungsform des erfindungsgemäßen Verfahrens dann eingesetzt werden, wenn die Katalysatoren als Formkörper eingesetzt werden. Werden demgemäß beispielsweise in einer ersten Zone Katalysator-Formkörper einer ersten Geometrie, in einer zweiten Zone Katalysator-Formkörper einer zweiten Geometrie und in einer dritten Zone Katalysator-Formkörper einer dritten Geometrie eingesetzt, so können sich die zur Abtrennung der Zonen beispielsweise bevorzugt eingesetzten Siebböden in der Maschenweite unterscheiden, die der Geometrie der jeweiligen Formkörper angepasst werden kann.

Ebenso können sich auch die Materialien, aus denen die mechanischen Trennvorrichtungen gefertigt sind, voneinander unterschieden.

Demgemäß beschreibt die vorliegende Erfindung auch ein Verfahren, wie oben beschrieben, das dadurch gekennzeichnet ist, dass die voneinander verschiedenen Katalysatoren durch mindestens eine mechanische Trennvorrichtung räumlich voneinander getrennt sind.

Die erfindungsgemäße Kompartimentierung in dem einzigen Reaktor kann auch ohne zusätzliche mechanische Trennvorrichtung realisiert werden. Dies ist beispielsweise durch eine spezielle Ausgestaltung der Innenwand oder der Innenwände des Reaktors möglich, die beispielsweise voneinander beabstandete Mulden, Ausnehmungen oder ähnliche Ausgestaltungen aufweisen, in die die voneinander verschiedenen Katalysatoren aufgenommen werden.

Ebenso ist es im Rahmen der erfindungsgemäßen Kompartimentierung auch möglich, dass die einzelnen Kompartimente mit den unterschiedlichen Zeolith-Katalysatoren direkt aneinandergrenzen, ohne dass die unterschiedlichen Zeolith-Katalysatoren voneinander beabstandet sind. So ist es möglich, den Reaktor in einer ersten Zone mit einem ersten Titanzeolith-Katalysator und in einer direkt angrenzenden zweiten Zone mit einem zweiten Titanzeolith-Katalysator, der sich vom ersten Zeolith-Katalysator unterscheidet, auszustatten, wobei die beiden Katalysatorzonen eine oder mehrere gemeinsame Grenzflächen aufweisen. An der Grenzfläche zwischen den beiden Zonen kann eine Durchmischung der beiden unterschiedlichen Zeolith-Katalysatoren stattfinden, solange gewährleistet ist, dass über die beiden Zonen hinweg eine Inhomogenität der Zeolith-Katalysatorverteilung vorliegt. Unter diesem Begriff der "Inhomogenität der Zeolith-Katalysatorverteilung" wird im Rahmen der vorliegenden Erfindung eine Ausführungsform verstanden, gemäß der mindestens ein Teil der ersten Zone vorwiegend, bevorzugt ausschließlich den ersten Zeolith-Katalysator und mindestens ein Teil der zweiten Zone vorwiegend, bevorzugt ausschließlich den zweiten Zeolith-Katalysator enthält.

Gemäß einer besonders bevorzugten Ausführungsform wird dies beispielsweise durch eine strukturierte Schüttung verschiedener Katalysatoren erreicht. Eine erste Zone des Reaktors wird dabei durch Schüttung mit einem ersten Titanzeolith-Katalysator ausgestattet, wodurch das erste Katalysator-Kompartiment hergestellt wird. Anschließend wird eine zweite Zone des Reaktors durch Aufschütten des zweiten, vom ersten verschiedenen Titanzeolith-Katalysator auf das erste Kompartiment unter Ausbildung des zweiten Kompartiments hergestellt. Ebenso können auch ein drittes oder weitere Kompartimente hinzugefügt werden, wobei im dritten oder einem weiteren Kompartiment jeweils einer der beiden ersten oder ein von den ersten beiden Katalysatoren verschiedener Zeolith-Katalysator verwendet werden kann. Diese Art der Herstellung wird im Rahmen der vorliegenden Erfindung als "strukturierte Schüttung" bezeichnet.

Diese strukturierte Schüttung bietet unter anderem im Vergleich zu herkömmlichen Verfahren, bei denen ein Reaktor mit nur einem einzigen Zeolith-Katalysator ausgestattet wird, den Vorteil, dass sich durch die gezielte Auswahl und damit Abfolge der Katalysatoren, die in verschiedenen Reaktorzonen eingesetzt werden, beispielsweise der Reaktionsumsatz positiv beeinflussen lässt. Beispielsweise bei kontinuierlicher Reaktionsführung, bei denen die Reaktionspartner Hydroperoxid und organische Verbindung durch den Reaktor geleitet werden und dabei die verschiedenen Reaktorzonen mit den unterschiedlichen Zeolith-Katalysatoren passieren, können die einzelnen Katalysatoren dem Reaktionsfortschritt angepasst werden.

So ist es beispielsweise möglich, in einer ersten Zone des Reaktors, in der die Konzentration der nicht umgesetzten Reaktionspartner hoch ist, den Titanzeolith-Katalysator so zu wählen, dass der Umsatz beispielsweise bei einer exothermen Reaktion gerade so hoch ist, dass die entstehende Wärme noch abgeführt werden kann. In einer nächsten Reaktorzone, in der die Konzentration der Reaktionspartner geringer ist, kann dann ein Titanzeolith-Katalysator eingesetzt werden, der beispielsweise einen höheren Umsatz gewährleistet, der also hinsichtlich der Reaktion aktiver ist. Die beim Durchgang der Reaktionspartner Hydroperoxid und organische Verbindung durch den Reaktor entstehende Inhomogenität in deren Konzentrationen und die dadurch resultierende Inhomogenität des Reaktionsgemisches, enthaltend die Reaktionspartner Hydroperoxid und organische Verbindung und das daraus entstehende Reaktionsprodukt oder die daraus entstehenden Reaktionsprodukte, können demgemäß durch eine entsprechende Wahl von unterschiedlichen Titanzeolith-Katalysatoren und damit durch eine Inhomogenität von beispielsweise der Katalysatoraktivität über den Reaktor hinweg kompensiert werden.

Beispielweise ist es möglich, dass bei der Umsetzung von Hydroperoxid mit organischer Verbindung beispielsweise Produkte entstehen, die in der Lage sind, wiederum mit entweder Hydroperoxid oder organischer Verbindung oder sowohl mit Hydroperoxid und organischer Verbindung zu einem unerwünschten Folgeprodukt weiterzureagieren. Beim Durchgang des Reaktionsgutes durch den Reaktor werden in diesem Fall die Konzentration an erwünschtem Produkt und damit die Wahrscheinlichkeit, dass sich unerwünschtes Folgeprodukt bildet, immer größer. Dementsprechend kann beispielsweise in einer ersten Reaktorzone ein erster Titanzeolith-Katalysator eingesetzt werden und in einer zweiten Reaktorzone ein anderer Titanzeolith-Katalysator eingesetzt werden, der die Umsetzung von Hydroperoxid und organischer Verbindung zwar noch katalysiert, hinsichtlich der Weiterreaktion zu unerwünschtem Folgeprodukt jedoch inaktiver ist als der Zeolith-Katalysator in der ersten Reaktorzone.

Dabei ist gemäß einer Ausführungsform in einem ersten Kompartiment des Reaktors einen Titanzeolithkatalysator mit einem hohen Titangehalt und demgemäß mit einer hohen Aktivität bezüglich der Umsetzung von organischer Verbindung mit Hydroperoxid und in einem zweiten Kompartiment des Reaktors einen Titanzeolithkatalysator mit einem im Vergleich zum ersten Titanzeolithkatalysator niedrigeren Titangehalt einzusetzen. Gemäß einer weiteren Ausführungsform weisen die beiden Titanzeolithkatalysatoren hierbei die gleiche Kristallstruktur auf. Gemäß einer weiteren Ausführungsform weisen die beiden Titanzeolithkatalysatoren unterschiedliche Kristallstruktur auf, wobei beispielsweise der Katalysator im ersten Kompartiment eine Kristallstruktur vom MFI-Typ und der Katalysator im zweiten Kompartiment eine Kristallstruktur vom MWW-Typ aufweist. Ebenso kann der Katalysator im ersten Kompartiment eine Kristallstruktur vom MWW-Typ und der Katalysator im zweiten Kompartiment eine Kristallstruktur vom MFI-Typ aufweisen. Im Rahmen dieser Ausführungsformen ist es bevorzugt, dass die Reaktionsmischung beim Durchgang durch den Reaktor zunächst das erste Kompartiment und anschließend das zweite Kompartiment durchläuft.

Demgemäß betrifft die vorliegende Erfindung auch ein Verfahren, wie oben beschrieben, das dadurch gekennzeichnet ist, dass die Umsetzung der organischen Verbindung in einem einzigen Reaktor durchgeführt wird.

Ebenso betrifft die vorliegende Erfindung auch einen kontinuierlich betriebenen isothermen Festbettrohrreaktor zur Umsetzung einer organischen Verbindung mit einem Hydroperoxid, umfassend mindestens zwei räumlich voneinander getrennte, voneinander verschiedene Titanzeolith-Katalysatoren, wobei sich die Titanzeolith-Katalysatoren im Titangehalt oder in der Zeolithstruktur oder sowohl im Titangehalt als auch in der Zeolithstruktur unterscheiden. Der Festbettrohrreaktor ist bevorzugt ein Festbettrohrreaktor zur Umsetzung von Propen mit Wasserstoffperoxid.

Die erfindungsgemäße Kompartimentierung kann im Rahmen der vorliegenden Erfindung beispielsweise auch durch den Einsatz von mindestens zwei seriell geschalteten Reaktoren realisiert werden, wobei in einem ersten Reaktor mindestens ein Titanzeolith-Katalysator zur Umsetzung verwendet wird und in mindestens einem weiteren Reaktor mindestens ein weiterer Titanzeolith-Katalysator zur Umsetzung zum Einsatz kommt, der sich von dem im ersten Reaktor eingesetzten Zeolith-Katalysator unterscheidet.

Im Rahmen dieser Ausführungsform ist es beispielsweise möglich, in mindestens einem ersten Reaktor einen ersten Katalysator und in mindestens einem zweiten Reaktor einen zweiten, vom ersten Katalysator verschiedenen Katalysator einzusetzen, wobei der Katalysator im ersten Reaktor beispielsweise als zeolithisches Material oder als Formkörper oder als Mischung aus zeolithischem Material und Formkörper und der Katalysator im zweiten Reaktor als zeolithisches Material oder als Formkörper oder als Mischung aus zeolithischem Material und Formkörper vorliegen können.

Ebenso kann der Katalysator beispielsweise als Netzkatalysator auf Basis von inerten Netzgeweben wie beispielsweise inerten Netzgeweben aus Metallen, Kunststoffen, Aluminiumoxiden, Glasfasern, Kohlefasern und/oder Graphit eingesetzt werden. Bezogen auf die Gewichtsmenge des Zeolithkatalysators weisen solche Netzkatalysatoren bevorzugt einen Alkalimetallgehalt von weniger als 500 ppm auf. Hergestellt werden diese Netzkatalysatoren bevorzugt gemäß einem Verfahren, bei dem mindestens ein Titanzeolith auf inertes Netzgewebe aufkristallisiert wird. Netzkatalysatoren dieser Art und Wege zu ihrer Herstellung sind in der der EP 0 883 439 B1 beschrieben, deren diesbezüglicher Inhalt in den Kontext der vorliegenden Erfindung durch Bezugnahme vollumfänglich einbezogen wird.

Demgemäß betrifft die vorliegende Erfindung auch einen seriell gekoppelten Reaktorverbund aus zwei seriell gekoppelten Reaktoren zur Umsetzung einer organischen Verbindung mit einem Hydroperoxid, wobei der erste Reaktor ein isothermer Festbettrohrreaktor und der zweite Reaktor ein adiabatischer Festbettrohrreaktor ist, wobei die zwei Reaktoren jeweils voneinander verschiedene Titanzeolith-Katalysatoren enthalten, und wobei sich die Titanzeolith-Katalysatoren im Titangehalt oder in der Zeolithstruktur oder sowohl im Titangehalt als auch in der Zeolithstruktur unterscheiden. Der Reaktorverbund ist bevorzugt ein Reaktorverbund zur Umsetzung von Propen mit Wasserstoffperoxid.

Unabhängig davon, ob im erfindungsgemäßen Verfahren einer oder mehrere Reaktoren zur Umsetzung der organischen Verbindung mit Hydroperoxid eingesetzt werden, werden die Reaktionsbedingungen in jedem Reaktor bevorzugt so gewählt, dass das Reaktionsgemisch einphasig und flüssig ist.

Hinsichtlich der im Rahmen der vorliegenden Erfindung einsetzbaren Titanzeolith-Katalysatoren existieren keine Beschränkungen, solange gewährleistet ist, dass die Katalysatoren die Umsetzung der organischen Verbindung mittels einem Hydroperoxid katalysieren.

Zeolithe sind bekanntermaßen kristalline Alumosilikate mit geordneten Kanal- und Käfigstrukturen, die Mikroporen aufweisen, die vorzugsweise kleiner als ungefähr 0,9 nm sind Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W. M. Meier, D. H. Olson und Ch. Baerlocher, "Atlas of Zeolithe Structure Types", Elsevier, 5. Auflage, Amsterdam 2001.

Es sind nun auch Zeolithe bekannt, die kein Aluminium enthalten und bei denen im Silikatgitter an Stelle des Si(IV) teilweise Titan als Ti(IV) steht. Diese Titanzeolithe, insbesondere solche mit einer Kristallstruktur von MFI-Typ, sowie Möglichkeiten zu Ihrer Herstellung sind beschrieben, beispielsweise in der EP-A 0 311 983 oder EP-A 405 978. Außer Silicium und Titan können solche Materialien auch zusätzliche Elemente wie z.B. Aluminium, Zirkonium, Zinn, Eisen, Kobalt, Nickel, Gallium, Bor oder geringe Menge an Fluor enthalten. In den in dem erfindungsgemäßen Verfahren vorzugsweise regenerierten Zeolith-Katalysatoren kann das Titan des Zeoliths teilweise durch Vanadium, Zirkonium, Chrom oder Niob oder ein Gemisch aus zwei oder mehr davon ersetzt sein. Das molare Verhältnis von Titan und/oder Vanadium, Zirkonium, Chrom oder Niob zur Summe aus Silicium und Titan und/oder Vanadium und/oder Zirkonium, und/oder Chrom und/oder Niob liegt in der Regel im Bereich von 0,01 : 1 bis 0,1 : 1.

Dabei sind im einzelnen Titan haltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI , MFS-, MON-, MOR-, MSO-, MTF-, MTN-, MTT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Titanzeolithe, insbesondere solche mit einer Kristallstruktur vom MFI-Typ, sowie Möglichkeiten zu ihrer Herstellung sind beispielsweise in der WO 98/55228, EP-A 0 311 983 oder der EP-A 0 405 978 beschrieben, deren diesbezüglicher Umfang vollumfänglich in den Kontext der vorliegenden Anmeldung einbezogen wird. Titanzeolithe mit beispielsweise einer Kristallstruktur vom MWW-Typ und Möglichkeiten zu ihrer Herstellung sind in der WO 02/28774 A2 oder in Wu et al., "Hydrothermal Synthesis of a novel Titanosilicate with MWW Topology", Chemistry Letters 2000, S. 774-775 beschrieben, deren diesbezüglicher Inhalt vollumfänglich in den Kontext der vorliegenden Anmeldung einbezogen wird. Beispielsweise sind konkrete Synthesen von Ti-MWW in den Beispielen 1 bis 5 der WO 02/28774 A2 beschrieben.

Generell wird das zeolithische Material nach der Abtrennung aus seiner Mutterlauge bei Temperaturen im Bereich von im allgemeinen 80 bis 160 °C, bevorzugt von 90 bis 145 °C und besonders bevorzugt von 100 bis 130 °C getrocknet und anschließend bevorzugt bei Temperaturen im Bereich von im allgemeinen 400 bis 750 °C, bevorzugt von 450 bis 600 °C und besonders bevorzugt von 490 bis 530 °C calciniert. Gemäß weiterer Ausführungsformen des erfindungsgemäßen Verfahrens wird der Zeolith nach der Abtrennung aus der Mutterlauge mit einer Wasser enthaltenden Zusammensetzung in Kontakt gebracht werden. Dieses Inkontaktbringen kann ebenso nach der Trocknung und/oder dem Calcinieren erstmals erfolgen oder wiederholt werden. In diesen Fällen können sich nach dem Inkontaktbringen mit der Wasser enthaltenden Zusammensetzung ein oder mehrere der oben beschriebenen Behandlung zur Aufkonzentrierung oder zum Abtrennen anschließen. Als Wasser enthaltende Zusammensetzung wird beispielsweise bevorzugt Wasser an sich verwendet. Ebenso sind wässrige Aminlösungen möglich, wobei das in dieser Lösung enthaltene mindestens eine Amin Ammoniak, ein organisches aliphatisches Amin oder ein quartäres Ammoniumhydroxid sein kann und wobei der Stickstoff in diesen Stickstoffverbindungen als Alkylreste beispielsweise Methyl-, Ethyl- oder Propylreste aufweisen kann und auch zwei oder mehr unterschiedliche Alkylreste an einem Stickstoff gebunden sein können. Das Inkontaktbringen mit beispielsweise bevorzugt Wasser an sich findet im allgemeinen bei Temperaturen im Bereich von Raumtemperatur bis 750 °C, bevorzugt von 100 bis 250 °C und besonders bevorzugt von 120 bis 175 °C statt, wobei das Inkontaktbringen bevorzugt zwischen 12 und 48 h andauert. Ganz besonders bevorzugt findet dieses Inkontaktbringen in einem Autoklaven statt.

Im Falle, dass der Zeolith nach dem Abtrennen aus der Mutterlauge getrocknet und/oder calciniert wurde und anschließend mit einer Wasser enthaltenden Zusammensetzung in Kontakt gebracht wurde, kann sich ein nochmaliges Trocknen und/oder Calcinieren anschließen. Diese Trocknung erfolgt dabei bei Temperaturen im Bereich von im allgemeinen 80 bis 160 °C, bevorzugt von 90 bis 145 °C und besonders bevorzugt von 100 bis 130 °C. Die anschließend bevorzugt erfolgende Calcinierung wird bei Temperaturen im Bereich von im allgemeinen 400 bis 750 °C, bevorzugt von 450 bis 600 °C und besonders bevorzugt von 490 bis 530 °C durchgeführt.

Zusätzlich oder statt des Inkontaktbringens mit der Wasser enthaltenden Zusammensetzung kann der Zeolith noch mit beispielsweise Wasserstoffperoxidlösung, unter anderem bevorzugt schwefelsaurer Wasserstoffperoxidlösung gewaschen werden. Ebenso ist es möglich, das zeolithische Material mit Alkalimetallionen zu behandeln, um den Zeolithen von der H-Form in die kationische Form zu bringen.

Titanzeolithe mit beispielsweise MWW- oder MPI-Struktur sind dafür bekannt, dass sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Titanzeolithe werden im Rahmen der vorliegenden Erfindung als Zeolith-Katalysatoren eingesetzt.

Demgemäß betrifft die vorliegende Erfindung ein Verfahren, wie oben beschrieben, das dadurch gekennzeichnet ist, dass die mindestens zwei, voneinander verschiedenen Zeolith-Katalysatoren Titanzeolith-Katalysatoren sind.

Gemäß einer Ausführungsform der vorliegenden Erfindung unterschieden sich die eingesetzten Zeolith-Katalysatoren durch ihren Titangehalt , gemäß derer zwei Titanzeolith-Katalysatoren mit unterschiedlichen Titangehalten eingesetzt werden.

Werden beispielsweise zwei Titanzeolith-Katalysatoren Typ mit unterschiedlichen Titangehalten eingesetzt, so weisen diese Titanzeolith-Katalysatoren die folgende chemische Zusammensetzung (I) oder (II)

x • TiO₂ (1-x) • SiO₂ (I)

wobei 0,0001 ≤ x ≤ 0,2, der

x • TiO₂ y • M₂O₃ (1-x-2y) • SiO₂ (II)

wobei 0,0001 ≤ x ≤ 0,2 und 0,0001 ≤ y ≤ 0,1 und M mindestens ein Element aus der Gruppe bestehend aus Aluminium, Bor, Chrom, Gallium, Germanium und Eisen ist. Die Variablen x und y bezeichnen hierbei den jeweiligen molaren Anteil.

Weitere Details hinsichtlich von Zeolith-Strukturtypen wie beispielsweise des Strukturtyps MFI, MEL oder des Strukturtyps MWW sind der oben genannten Literaturstelle W.M. Meier, D.H. Olson und Ch. Baerlocher "Atlas of Zeolite Structure Types", Elsevier, 5. Aufl., S. 202 und 203, Amsterdam 2001 zu entnehmen.

Demgemäß beschreibt die vorliegende Erfindung gemäß einer Ausführungsform ein Verfahren, wie oben beschrieben, das dadurch gekennzeichnet ist, dass sich die voneinander verschiedenen Titanzeolith-Katalysatoren in ihrem Titangehalt unterscheiden.

Bevorzugt werden im Rahmen der vorliegenden Erfindung Ti-Zeolithe mit MFI-Struktur, MEL-Struktur, MFI/MEL-Mischstruktur oder MWW-Struktur eingesetzt. Als weiterhin bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu beta-Zeolith isomorphen Gerüststruktur zu nennen. Ganz besonders bevorzugt werden im Rahmen der vorliegenden Erfindung Zeolith-Katalysatoren der TS-1-Struktur und der Ti-MWW-Struktur eingesetzt.

Gemäß einer weiteren Ausführungsform werden als voneinander verschiedene Titanzeolith-Katalysatoren solche eingesetzt, die sich in ihrem Strukturtyp unterscheiden. Hinsichtlich der unter anderem möglichen Strukturtypen sei auf die oben genannten Strukturtypen verwiesen.

Als beispielsweise bevorzugte Zeolith-Katalysatoren, die sich in ihrer Struktur unterscheiden, seien beispielsweise Zeolith-Katalysatoren vom TS-1-Typ und vom Ti-MWW-Typ zu nennen.

Demgemäß betrifft die vorliegende Erfindung ein Verfahren, wie oben beschrieben, das dadurch gekennzeichnet ist, dass sich die Titanzeolith-Katalysatoren im Titangehalt oder in der Zeolithstruktur oder sowohl im Titangehalt als auch der Zeolithstruktur unterscheiden.

Gemäß eines bevorzugten Verfahrens der vorliegenden Erfindung wird als Katalysator ein Titansilikalit-Katalysator, bevorzugt von Strukturtyp TS-1 eingesetzt. Voneinander unterschiedliche Katalysatoren sind demgemäß Katalysatoren, die sich beispielsweise im Titangehalt oder hinsichtlich des Porenvolumens oder der spezifischen Oberfläche oder des Titangehalts und des Porenvolumens oder des Titangehalts und der spezifischen Oberfläche oder des Titangehalts und der spezifischen Oberfläche und des Porenvolumens unterscheiden. Werden im erfindungsgemäßen Verfahren aus dem Titansilikalit-Material Katalysatorformkörper hergestellt, so unterscheiden sich voneinander verschiedene Formkörper in der Art des verwendeten zeolithischen Materials oder des Titangehalts oder des SiO₂-Gehalts oder durch die spezifische Oberfläche oder durch das Porenvolumen oder durch das Schüttgewicht oder durch die Härte wie beispielsweise die Seitendruckfestigkeit oder durch zwei oder mehr dieser Parameter. Beispielsweise können daher in einem einzigen Reaktor zwei verschiedene Formkörper verwendet werden, die unterschiedliche Titansilikalitmaterialien oder gleiche Titansilikalitmaterialien enthalten. Im Falle, dass sie gleiche Titansilikalitmaterialien enthalten, unterscheiden sich die Formkörper im Gehalt an Titansilikalit. Im Falle, dass sie unterschiedliche Titansilikalitmaterialien enthalten, können sich die Formkörper zusätzlich im Gehalt an Titansilikalit und/oder mindestens einem der oben genannten Parameter unterscheiden.

Unter den Umsetzungen von organischen Verbindungen mit Hydroperoxiden, die im erfindungsgemäßen Verfahren möglich sind, seien beispielhaft die folgenden genannt:
- die Epoxidation von Olefinen wie z.B. die Herstellung von Propenoxid aus Propen und H₂O₂ oder aus Propen und Gemischen, die H₂O₂ in situ liefern;
- Hydroxylierungen wie z.B die Hydroxylierung mono-, bi- oder polycyclischer Aromaten zu mono-, di- oder höher substituierten Hydroxyaromaten, beispielsweise die Umsetzung von Phenol und H₂O₂ oder von Phenol und Gemischen, die H₂O₂ in situ liefern, zu Hydrochinon;
- die Oximbildung aus Ketonen unter Anwesenheit von H₂O₂ oder Gemischen, die H₂O₂ in situ liefern, und Ammoniak (Ammonoximierung), beispielsweise die Herstellung von Cyclohexanonoxim aus Cyclohexanon;
die Baeyer-Villiger-Oxidation.

Bevorzugt werden im erfmdungsgemäßen Verfahren organische Verbindungen umgesetzt, die mindestens eine C-C-Doppelbindung aufweisen. Demgemäß beschreibt die vorliegende Erfindung ein Verfahren, wie oben beschrieben, das dadurch gekennzeichnet ist, dass die organische Verbindung mindestens eine C-C-Doppelbindung aufweist.

Als Beispiele für solche organischen Verbindungen mit mindestens einer C-C-Doppelbindung seien folgende Alkene genannt:
Ethen, Propen, 1-Buten, 2-Buten, Isobuten, Butadien, Pentene, Piperylen, Hexene, Hexadiene, Heptene, Octene, Diisobuten, Trimethylpenten, Nonene, Dodecen, Tridecen, Tetra- bis Eicosene, Tri- und Tetrapropen, Polybutadiene, Polyisobutene, Isoprene, Terpene, Geraniol, Linalool, Linalylacetat, Methylencyclopropan, Cyclopenten, Cyclohexen, Norbomen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren wie z.B. Acrylsäure, Methacrylsäure, Crotonsäure, Maleinsäure, Vinylessigsäure, ungesättigte Fettsäuren, wie z.B. Ölsäure, Linolsäure, Palmitinsäure, natürlich vorkommende Fette und Öle.

Bevorzugt werden im erfindungsgemäßen Verfahren Alkene verwendet, die 2 bis 8 Kohlenstoffatome enthalten. Besonders bevorzugt werden Ethen, Propen, und Buten umgesetzt. Insbesondere bevorzugt wird Propen umgesetzt.

Demgemäß betrifft die vorliegende Erfindung auch ein Verfahren, wie oben beschrieben, das dadurch gekennzeichnet ist, dass als organische Verbindung ein Alken eingesetzt wird.

Im erfindungsgemäßen Verfahren wird zur Umsetzung der organischen Verbindung mindestens ein Hydroperoxid eingesetzt. Im Rahmen der vorliegenden Anmeldung wird unter dem Begriff "Hydroperoxid" eine Verbindung der allgemeinen Formel ROOH verstanden. Details bezüglich der Hydroperoxidherstellung und von unter anderem im Rahmen des erfindungsgemäßen Verfahrens einsetzbaren Hydroperoxiden sind in der DE 198 35 907 A zu entnehmen, deren diesbezüglicher Inhalt in den Kontext der vorliegenden Anmeldung aufgenommen wird. Beispiele für erfindungsgemäß einsetzbare Hydroperoxide sind unter anderem tert-Butylhydroperoxid, Ethylbenzolhydroperoxid, tert-Amylhydroperoxid, Cumenhydroperoxid, Cyclohexylhydroperoxid, Methycyclohexylhydroperoxid, Tetrahydronaphthalinhydroperoxid, Isobutylbenzolhydroperoxid, Ethylnaphthalinhydroperoxid, Persäuren wie beispielsweise Peressigsäure oder Wasserstoffperoxid. Auch Gemische aus zwei oder mehr Hydroperoxiden sind erfindungsgemäß einsetzbar. Bevorzugt wird im Rahmen der vorliegenden Erfindung Wasserstoffperoxid als Hydroperoxid eingesetzt, wobei weiter bevorzugt eine wässrige Wasserstoffperoxidlösung eingesetzt wird.

Daher beschreibt die vorliegende Erfindung auch ein Verfahren zur katalytischen Epoxidierung eines Alkens, bevorzugt Propen, mit einem Hydroperoxid, bevorzugt Wasserstoffperoxid, in mindestens einem Reaktor unter Verwendung von mindestens zwei voneinander verschiedenen Titanzeolith-Katalysatoren, das dadurch gekennzeichnet ist, dass mindestens zwei der voneinander verschiedenen Zeolith-Katalysatoren räumlich voneinander getrennt eingesetzt werden.

Beispielsweise bevorzugt ist die räumliche Trennung über den Einsatz von 2 Reaktoren in serieller Kopplung und/oder über strukturierte Schüttung realisiert.

Als Lösungsmittel können prinzipiell alle für die jeweilige Umsetzung geeigneten Lösungsmittel eingesetzt werden. Unter anderem bevorzugt sind beispielsweise
- Wasser,
- Alkohole, bevorzugt niedere Alkohole, weiter bevorzugt Alkohole mit weniger als 6 Kohlenstoffatomen wie beispielsweise Methanol, Ethanol, Propanole, Butanole, Pentanole,
- Diole oder Polyole, bevorzugt solche mit weniger als 6 Kohlenstoffatomen,
- Ether wie beispielsweise Diethylether, Tetrahydrofuran, Dioxan, 1,2-Diethoxyethan, 2-Methoxyethanol,
- Ester wie beispielsweise Methylacetat oder Butyrolacton,
- Amide wie beispielsweise Dimethylformamid, Dimethylacetamid, N-Methylpyrrolidon,
- Ketone wie beispielsweise Aceton,
- Nitrile wie beispielsweise Acetonitril
- oder Gemische aus zwei oder mehr der vorgenannten Verbindungen.

Besonders bevorzugt zur Umsetzung von Propen sind beispielsweise Wasser, Methanol, Acetonitril oder Gemische aus Wasser und Methanol oder Wasser und Acetonitril.

Wird ein wie oben beschriebenes, beispielsweise bevorzugtes Verfahren in mindestens zwei Reaktoren durchgeführt, so kann beispielsweise in einem ersten Reaktor die Umsetzung in Anwesenheit eines ersten Katalysators vom TS-1-Typ und in einem zweiten Reaktor in Anwesenheit eines zweiten Katalysators vom MWW-Typ durchgeführt werden. Hierbei ist es beispielsweise bevorzugt, im ersten Reaktor als Lösungsmittel Methanol oder ein Methanol-Wasser-Gemisch und im zweiten Reaktor als Lösungsmittel Acetonitril oder ein Acetonitril-Wassergemisch zu verwenden. Ebenso kann im ersten Reaktor der Katalysator vom MWW-Typ eingesetzt werden und im zweiten Reaktor der Katalysator vom TS-1-Typ. In jedem dieser Fälle ist der Katalysator vom MWW-Typ bevorzugt ein Ti-MWW-Katalysator.

Demgemäß betrifft die vorliegende Erfindung auch ein Verfahren, wie oben beschrieben, das dadurch gekennzeichnet ist, dass die Umsetzung in mindestens zwei seriell geschalteten Reaktoren durchgeführt wird, wobei in mindestens einem ersten Reaktor mindestens ein Titanzeolith-Katalysator und in mindestens einem zweiten Reaktor mindestens ein weiterer Titanzeolith-Katalysator eingesetzt wird, der sich von mindestens einem der in dem ersten Reaktor eingesetzten Titanzeolith-Katalysatoren unterscheidet.

Zwischen den erfindungsgemäß seriell gekoppelten Reaktoren kann erfindungsgemäß mindestens eine Zwischenabtrennung vorgesehen werden. In dieser Zwischenabtrennung wird beispielsweise bevorzugt mindestens ein Reaktionsprodukt aus dem Reaktionsgemisch, das den ersten Reaktor verlässt, abgetrennt. Ebenso kann mindestens ein Nebenprodukt oder mindestens ein Folgeprodukt abgetrennt werden. Ebenso kann eine Mischung, enthaltend mindestens zwei der oben genannten Produkte, abgetrennt werden. Bein einem unter anderem bevorzugten Verfahren, bei dem ein Alken mit einem Hydroperoxid umgesetzt wird, wird in einer solchen Zwischenabtrennung bevorzugt nichtumgesetztes Hydroperoxid abgetrennt. Eine erfindungsgemäße zweistufige Umsetzung von Alken mit einem Hydroperoxid unter Verwendung einer Zwischenabtrennung umfasst daher beispielsweise folgende Stufen (A) bis (C):
(A) Umsetzung von Alken mit einem Hydroperoxid in mindestens einem Lösungsmittel oder Lösungsmittelgemisch in Anwesenheit eines ersten Titanzeolith-Katalysators unter Erhalt einer Mischung, enthaltend Alkenoxid und nicht umgesetztes Hydroperoxid;
(B) Abtrennung des nicht-umgesetzten Hydroperoxids aus der aus (A) resultierenden Mischung;
(C) Umsetzung des abgetrennten Hydroperoxids aus (B) mit Alken in mindestens einem Lösungsmittel oder Lösungsmittelgemisch in Anwesenheit eines zweiten Titanzeolith-Katalysators.

Demgemäß beschreibt die vorliegende Erfindung auch ein Verfahren, wie oben beschrieben, das dadurch gekennzeichnet ist, dass zwischen mindestens zwei der seriell geschalteten Reaktoren eine Zwischenabtrennung der umgesetzten organischen Verbindung erfolgt.

Die Abtrennung des eingesetzten Hydroperoxids gemäß (B) kann gemäß sämtlicher geeigneter Verfahren erfolgen. Die Abtrennung des beispielsweise bevorzugt eingesetzten Wasserstoffperoxids erfolgt bevorzugt destillativ, wobei eine oder mehrere, bevorzugt eine Destillationskolonne eingesetzt wird.

In einem erfindungsgemäßen Verfahren, umfassend die Stufen (A) bis (C), wird beispielsweise besonders bevorzugt Propen mit Wasserstoffperoxid umgesetzt, wobei gemäß (A) als Zeolith-Katalysator bevorzugt ein Katalysator vom TS-1-Typ und als Lösungsmittel bevorzugt Methanol und gemäß (C) als Zeolith-Katalysator bevorzugt ein Katalysator vom Ti-MWW-Typ und als Lösungsmittel bevorzugt Acetonitril eingesetzt werden.

In einem erfindungsgemäßen Verfahren, umfassend die Stufen (A) bis (C), wird beispielsweise besonders bevorzugt auch Propen mit Wasserstoffperoxid umgesetzt, wobei gemäß (A) als Zeolith-Katalysator bevorzugt ein Katalysator vom Ti-MWW-Typ und als Lösungsmittel bevorzugt Acetonitril und gemäß (C) als Zeolith-Katalysator bevorzugt ein Katalysator vom TS-1-Typ und als Lösungsmittel bevorzugt Methanol eingesetzt werden.

Die Reaktionsbedingungen, unter denen die Umsetzung in den jeweiligen Reaktoren durchgeführt werden, können grundsätzlich den jeweiligen Anforderungen des Verfahrens angepasst werden. Gleiches gilt beispielsweise für die genaue Größe einzelner Katalysator-Kompartimente in den jeweiligen Reaktoren beziehungsweise für die Verhältnisse der Geometrien wie Längen, Durchmesser oder Volumina dieser Kompartimente untereinander oder die in den jeweiligen Reaktoren eingesetzten Mengen der entsprechenden Katalysatoren oder für die Titangehalte der voneinander verschiedenen Katalysatoren.

Die Reaktoren können erfindungsgemäß sowohl in Suspensions- als auch in Festbettfahrweise betrieben werden. In Suspensionsfahrweise wird besonders bevorzugt in der Ausführungsform der seriell gekoppelten Reaktoren gearbeitet, wobei beispielsweise in einem ersten Reaktor eine Suspension eines ersten Zeolith-Katalysators und in einem zweiten, mit dem ersten Reaktor seriell gekoppelten Reaktor eine Suspension eines zweiten Zeolith-Katalysators eingesetzt wird. Ebenso ist es möglich, mindestens einen Reaktor in Suspensionfahrweise und mindestens einen Reaktor in Festbettfahrweise zu betreiben.

Gemäß einer bevorzugten Ausführungsform des erfindungsgemäßen Verfahrens findet die Umsetzung der organischen Verbindung mit dem Hydroperoxid in mindestens einem Festbettreaktor statt. Erfolgt die Umsetzung in einem einzigen Reaktor, so ist dieser ein Festbettreaktor, bevorzugt ein kontinuierlich betriebener Festbettreaktor, weiter bevorzugt ein kontinuierlich betriebener Festbettrohrreaktor und ganz besonders bevorzugt ein kontinuierlich betriebener isothermer Festbettrohrreaktor. Findet die Umsetzung beispielsweise in zwei seriell gekoppelten Reaktoren statt, so findet die erste Umsetzung bevorzugt in einem isothermen Festbettrohrreaktor und die zweite Umsetzung bevorzugt in einem adiabatischen Festbettrohrreaktor statt. Demgemäß werden die Umsetzungen gemäß (A) und (C) in jeweils einem Festbettreaktor und besonders bevorzugt in jeweils einem Festbettrohrreaktor durchgeführt. Insbesondere bevorzugt werden die Umsetzung gemäß (A) in einem isothermen Festbettrohrreaktor und die Umsetzung gemäß (C) in einem adiabatischen Festbettrohrreaktor durchgeführt.

Daher betrifft die vorliegende Erfindung auch ein Verfahren, wie oben beschrieben, das dadurch gekennzeichnet ist, dass mindestens einer der eingesetzten Reaktoren in Festbettfahrweise betrieben wird.

Die im Verfahren verwendeten Zeolith-Katalysatoren können nach der Reaktion entweder im Reaktor oder außerhalb des Reaktors oder sowohl im Reaktor als auch außerhalb des Reaktors gemäß einem oder mehreren geeigneten Verfahren regeneriert werden. Gemäß einem bevorzugten Verfahren werden die Zeolith-Katalysatoren in dem Reaktor, in dem die Umsetzung des Propens erfolgt, mittels einer thermischen Behandlung der Zeolith-Katalysatoren in Gegenwart eines Gasstroms bei Temperaturen oberhalb von 120°C, vorzugsweise oberhalb von 350°C und insbesondere bei 400°C bis 650°C regeneriert, wobei während der thermischen Behandlung die massenbezogene Verweilzeit des Gasstroms über dem Katalysator mehr als 2 Stunden beträgt, bevorzugt im Bereich von 3 bis 10 Stunden und insbesondere bevorzugt im Bereich von 4 bis 6 Stunden liegt. Das Regeneriergas enthält hierbei im Allgemeinen weniger als 20 Vol.-%, bevorzugt 0,1 bis 10 Vol.-%, insbesondere 0,1 bis 5 Vol.-% und noch weiter bevorzugt 0,1 bis 2 Vol.-% Sauerstoff. Vorzugsweise wird ein Gemisch aus Luft und entsprechenden Volumina Stickstoff eingesetzt. Der erfindungsgemäß verwendete Begriff "massenbezogene Verweilzeit" bezeichnet dabei das Verhältnis der Katalysator-Masse (M_{Kat}), dividiert durch den Massenstrom (M_{Gase}) des bei der Regenerierung verwendeten Gases. Dabei wird im allgemeinen so gefahren, dass der Druckverlust über dem Reaktor nicht mehr als 4 bar, vorzugsweise nicht mehr als 3 bar und insbesondere nicht mehr als 2,5 bar beträgt.

Wie bereits oben erwähnt, werden gemäß einer bevorzugten Ausführungsform der vorliegenden Erfindung die Reaktoren, in denen die Umsetzung der organischen Verbindung mit dem Hydroperoxid erfolgt, mit Zeolith-Katalysatorformkörpern ausgestattet. Die Herstellung der Katalysatorformkörper kann hierbei generell gemäß sämtlicher geeigneter Verfahren durchgeführt werden. Was den spezifischen Schritt der Herstellung eines Formkörpers anbelangt, so sei auf die WO 98/55229 und die DE 102 32 406.9 verwiesen, deren diesbezüglicher Inhalt durch Bezugnahme in den Kontext der vorliegenden Anmeldung einbezogen wird.

Bevorzugt wird das zeolithische Material, das aus seiner Mutterlauge abgetrennt und gegebenenfalls mindestens einer Behandlung wie Waschen, Trocknen, Calcinieren, Inkontaktbringen mit einer Wasser enthaltenden Zusammensetzung oder Behandeln mit Wasserstoffperoxidlösung unterworfen wurde, mit mindestens einem Bindemittel versetzt. Weitere Zusatzstoffe wie beispielsweise Mischungen aus Wasser und mindestens einem Alkohol oder mindestens eine viskositätssteigernde organische Verbindung oder mindestens eine porenbildende Verbindung, wie sie aus dem Stande der Technik bekannt sind, können ebenfalls zugegeben werden.

Als Bindemittel kann im Wesentlichen jede Verbindung eingesetzt werden, die die Kohäsion zwischen den Teilchen des zeolithischen Materials erhöht. Bevorzugte Bindemittel sind solche aus der Gruppe bestehend aus hydratisiertem Kieselgel, Kieselsäure, Kieselgel, Tetraalkoxysilikaten, Tetraalkoxytitanaten, Tetraalkoxyzirkonaten und Mischungen aus zwei oder mehr davon. Besonders bevorzugt sind Tetramethoxysilikat, Tetraethoxysilikat, Tetrapropoxysilikat, Tetrabutoxysilikat oder Kieselsol. Besonders bevorzugt sind Tetramethoxysilikat, Tetraethoxysilikat und Kieselsol, wobei insbesondere bevorzugt Kieselsol ist. Weitere Bindemittel sind etwa Aluminiumoxid oder Calciumphosphat oder Mischungen aus zwei oder mehr der genannten Bindemittel.

Weitere Bindemittel sind in der WO 98/55229 und der DE 102 32 406.9 beschrieben, deren diesbezüglicher Inhalt durch Bezugnahme in den Kontext der vorliegenden Anmeldung einbezogen wird.

Die genannten Bindemittel sind entweder allein oder als Mischungen aus zwei oder mehr davon einsetzbar. Weitere Bindemittel wie Oxide des Siliciums, Bors, Phosphors, Zirkoniums und/oder Titans können zusätzlich verwendet werden.

Im Rahmen der erfindungsgemäßen Herstellung des Formkörpers werden im allgemeinen bis zu 80 Gew.-%, bevorzugt 10 bis 80 Gew.-%, weiter bevorzugt 10 bis 75 Gew.-% und besonders bevorzugt 20 bis 40 Gew.-% Bindemittel, bezogen auf das Gesamtgewicht des resultierenden Formkörpers, eingesetzt.

Gemäß einer weiter bevorzugten Ausführungsform des erfindungsgemäßen Verfahrens wird dem zeolithischen Material mindestens ein Porenbildner zugefügt. Bevorzugt werden hierbei Polymere und weiter bevorzugt Polymere, die in Wasser oder wässrigen Lösungsmitteln dispergierbar, emulgierbar oder suspendierbar sind, verwendet. Dieses mindestens eine Polymer wird bevorzugt ausgewählt aus der Gruppe bestehend aus Vinylpolymeren wie beispielsweise Polystyrol, Polyacrylaten, Polymethacrylaten, Polyolefinen, Polyamiden und Polyestern. Diese porenbildenden Polymere werden nach der Herstellung der Formkörper durch Calcinieren bei geeigneten Temperaturen aus dem Formkörper entfernt. Werden Polymere als Porenbildner zugegeben, so werden diese in einem Anteil von im allgemeinen 5 bis 50 Gew.-%, bevorzugt 7 bis 35 Gew.-% und besonders bevorzugt von 10 bis 20 Gew.-%, jeweils bezogen auf das Gesamtgewicht der anorganischen Anteile aus Bindemittel und Zeolith, zugegeben.

Weiter bevorzugt wird mindestens ein Verstrangungshilfsmittel zugegeben. Als Verstrangungshilfsmittel kann im wesentliches jede Verbindung eingesetzt werden, die zur Verbesserung der Misch-, Knet- oder Fließeigenschaften führt. Bevorzugt sind organische hydrophile Polymere wie beispielsweise Cellulose, Cellulosederivate wie beispielsweise Alkylcellulosen, Stärke, Polyacrylate, Polymethacrylate, Polyvinylalkohol, Polyvinylpyrrolidon, Polyisobuten oder Polytetrahydrofuran. Erfindungsgemäß werden diese Verbindungen auch deshalb eingesetzt, weil sie die mechanische Stabilität der Formkörper, bevorzugt beim Formen und Trocknen und bei der anschließenden Verwendung als Katalysatorformkörper, erhöhen. Diese Verbindungen werden aus dem Formkörper durch Calcinieren bei geeigneten Temperaturen entfernt.

Weitere Zusatzstoffe sind in EP 0 389 041 A, EP 0 200 260 A und in WO 95/19222 beschrieben, deren diesbezüglicher Inhalt durch Bezugnahme in den Kontext der vorliegenden Anmeldung einbezogen wird.

Gemäß einer bevorzugten Ausführungsform des erfindungsgemäßen Verfahrens wird nach der Zugabe des mindestens einen Bindemittels zum zeolithischen Material die mindestens eine organische viskositätssteigernde Verbindung zugegeben und die erhaltene Masse im Bereich von 10 bis 180 min. in einer Knetvorrichtung oder einem Extruder homogenisiert. Diese Homogenisierung erfolgt bei Temperaturen, die im Allgemeinen etwa 10 °C unter dem Siedepunkt des Verstrangungshilfsmittels liegen. Dabei wird im Allgemeinen ein Druck angewandt, der in etwa bei Umgebungsdruck oder bei leichtem Überdruck liegt.

Gemäß einer weiteren bevorzugten Ausführungsform des erfindungsgemäßen Verfahrens wird während des Knetvorganges zum zeolithischen Material zunächst die mindestens eine porenbildende Verbindung und anschließend das mindestens eine Bindemittel zugegeben, woraufhin in einem oder auch mehreren Schritten die mindestens eine Wasser enthaltende Zusammensetzung, bevorzugt Wasser, zugegeben wird. Gemäß einer weiter bevorzugten Ausführungsform wird zunächst während des Knetens die mindestens eine porenbildende Verbindung und anschließend ein Teil des mindestens einen Bindemittels zugegeben, woraufhin in einem oder auch mehreren Schritten ein Teil der mindestens einen Wasser enthaltenden Zusammensetzung, bevorzugt Wasser, zugegeben wird. Im Anschluss daran wird der restliche Teil des Bindemittels und daraufhin in einem oder mehreren Schritten der Rest der mindestens einen Wasser enthaltenden Zusammensetzung, bevorzugt Wasser, zugegeben.

Bevorzugt werden dem zeolithischen Material Kieselsol und/oder eine Polystyroldispersion und/oder Cellulose und/oder ein Cellulosederivat wie beispielsweise Alkylcellulose und/oder Polyethylenoxid und/oder Wasser zugegeben. Beispielsweise bevorzugt wird das zeolithische Material mit Kieselsol, einer Polystyroldispersion, Methylcellulose und Wasser versetzt und dann in einer geeigneten Vorrichtung durch Kneten homogenisiert.

Demgemäß beschreibt die vorliegende Erfindung auch ein Verfahren, wie oben beschrieben, das dadurch gekennzeichnet ist, dass die Herstellung des Formkörpers gemäß (b) mindestens den Schritt (aa) umfasst:
(aa) Kneten des gemäß (a) erhaltenen porösen oxidischen Materials unter Zugabe mindestens eines Bindemittels oder mindestens eines Verstrangungshilfsmittels oder mindestens eines Porenbildners oder einer Wasser enthaltenden Zusammensetzung oder eines Gemischs aus zwei oder mehr davon.

Gemäß einer weiter bevorzugten Ausführungsform wird nach dem Kneten der Masse, wie oben beschrieben, die derart geknetete Masse zu einem Formkörper verformt. Dies kann generell gemäß sämtlicher geeigneter Verfahren erfolgen. Bevorzugt werden im erfindungsgemäßen Verfahren die Formkörper mittels eines Extruders hergestellt. Wiederum bevorzugt werden hierbei Extrudate hergestellt, die einen Durchmesser im Bereich von 1 bis 10 mm, weiter bevorzugt von 1 bis 5 mm und insbesondere bevorzugt von 1 bis 2 mm aufweisen.

Die Verformung kann bei Umgebungsdruck oder bei im Vergleich zum Umgebungsdruck erhöhten Druck im Bereich von im allgemeinen 1 bis 700 bar erfolgen. Weiterhin kann die Verformung bei Umgebungstemperatur oder bei im Vergleich zur Umgebungstemperatur erhöhter Temperatur im Bereich von im allgemeinen 20 bis 300 °C erfolgen. Weiter kann die Verformung in kontrollierter Atmosphäre durchgeführt werden, wobei generell inerte Gasatmosphären, reduzierende oder oxidierende Atmosphären möglich sind.

Die Abtrennung der einzelnen Formkörper aus dem aus dem Extruder austretenden Formmassenstrang kann generell gemäß sämtlicher möglicher Verfahren erfolgen. Besonders bevorzugt wird der pastöse Formmassenstrang im Extruder dadurch abgetrennt, dass zum Zertrennen die pastöse Formmasse mit mindestens einem Strom, enthaltend mindestens ein fluides Medium, in Kontakt gebracht wird. Weiter bevorzugt ist das fluide Medium ein Gas oder eine Flüssigkeit, insbesondere bevorzugt im wesentlichen Luft. Ebenfalls weiter bevorzugt wird der Strang der pastösen Formmasse periodisch zertrennt. Durch dieses Verfahren ist es möglich, im Vergleich zu den mechanischen Abtrennverfahren des Standes der Technik Formkörper herzustellen, die eine höhere Schüttdichte aufweisen. Dies wirkt sich insbesondere bei der Verwendung in Festbettreaktoren vorteilhaft aus.

Demgemäß beschreibt die vorliegende Erfindung auch ein Verfahren, wie oben beschrieben, das dadurch gekennzeichnet ist, dass die Herstellung des Formkörpers gemäß (b) mindestens die Schritte (aa) und (bb) umfasst:
(aa) Kneten des gemäß (a) erhaltenen porösen oxidischen Materials unter Zugabe mindestens eines Bindemittels oder mindestens eines Verstrangungshilfsmittels oder mindestens eines Porenbildners oder einer Wasser enthaltenden Zusammensetzung oder einem Gemisch aus zwei oder mehr davon;
(bb) Verformen der gemäß (aa) erhaltenen gekneteten Mischung unter Erhalt mindestens eines Formkörpers.

Weiter bevorzugt werden die Formkörper daraufhin bei Temperaturen im Bereich von 30 bis 140 °C, bevorzugt von 60 bis 135 °C und besonders bevorzugt von 90 bis 130 °C getrocknet, wobei die Trocknungszeiträume im allgemeinen im Bereich von 1 bis 20 h, bevorzugt im Bereich von 2 bis 10 h und besonders bevorzugt im Bereich von 3 bis 5 h liegen. Dabei werden Aufheizraten von im allgemeinen 0,5 bis 5 °C/min, bevorzugt von 1 bis 4 °C/min und besonders bevorzugt von 1,5 bis 3 °C/min gewählt.

Demgemäß beschreibt die vorliegende Erfindung auch ein Verfahren, wie oben beschrieben, das dadurch gekennzeichnet ist, dass die Herstellung des Formkörpers gemäß (b) mindestens die Schritte (aa) bis (cc) umfasst:
(aa) Kneten des gemäß (a) erhaltenen porösen oxidischen Materials unter Zugabe mindestens eines Bindemittels oder mindestens eines Verstrangungshilfsmittels oder mindestens eines Porenbildners oder einer Wasser enthaltenden Zusammensetzung oder einem Gemisch aus zwei oder mehr davon;
(bb) Verformen der gemäß (aa) erhaltenen gekneteten Mischung unter Erhalt mindestens eines Formkörpers;
(cc) Trocknen des gemäß (bb) erhaltenen Formkörpers.

Weiter bevorzugt werden die getrockneten Formkörper daraufhin bei Temperaturen im Bereich von 400 bis 800 °C, bevorzugt von 425 bis 600 °C und besonders bevorzugt von 450 bis 500 °C calciniert, wobei die Calcinierungszeiträume im allgemeinen im Bereich von 1 bis 20 h, bevorzugt im Bereich von 2 bis 10 h und besonders bevorzugt im Bereich von 3 bis 7 h liegen. Dabei werden Aufheizraten von im allgemeinen 0,25 bis 2 °C/min, bevorzugt von 0,5 bis 1,5 °C/min und besonders bevorzugt von 0,75 bis 1,25 °C/min gewählt. Ganz besonders bevorzugt wird der getrocknete Formkörper unter Luft und/oder Magerluft calciniert.

Demgemäß beschreibt die vorliegende Erfindung auch ein Verfahren, wie oben beschrieben, das dadurch gekennzeichnet ist, dass die Herstellung des Formkörpers gemäß (b) mindestens die Schritte (aa) bis (dd) umfasst:
(aa) Kneten des gemäß (a) erhaltenen porösen oxidischen Materials unter Zugabe mindestens eines Bindemittels oder mindestens eines Verstrangungshilfsmittels oder mindestens eines Porenbildners oder einer Wasser enthaltenden Zusammensetzung oder einem Gemisch aus zwei oder mehr davon;
(bb) Verformen der gemäß (aa) erhaltenen gekneteten Mischung unter Erhalt mindestens eines Formkörpers;
(cc) Trocknen des gemäß (bb) erhaltenen Formkörpers;
(dd) Calcinieren des gemäß (cc) erhaltenen getrockneten Formkörpers.

Vor oder nach dem Trocknen und/oder Calcinieren der gemäß (bb) erhaltenen Formkörper können diese, wie oben hinsichtlich des zeolithischen Materials beschrieben, mit einer Wasser enthaltenden Zusammensetzung in Kontakt gebracht werden. Werden die Formkörper nach dem Trocknen und/oder Calcinieren mit der Wasser enthaltenden Zusammensetzung in Kontakt gebracht, so schließt sich bevorzugt ein nochmaliges Trocknen und/oder Calcinieren an, das gemäß der unter (cc) und/oder (dd) beschriebenen Verfahrensführungen durchgeführt wird.

Demgemäß beschreibt die vorliegende Erfindung auch einen Katalysatorformkörper, wie oben beschrieben, erhältlich gemäß einen Verfahren, umfassend die Stufe (aa) oder die Stufen (aa) und (bb) oder die Stufen (aa), (bb) und (cc) oder die Stufen (aa), (bb), (cc) und (dd), dadurch gekennzeichnet, dass nach Stufe (bb) oder Stufe (cc) oder Stufe (dd) der Formkörper mit einer Wasser enthaltenden Zusammensetzung in Kontakt gebracht wird.

### Beispiele

Sämtliche Beispiele wurden in einem Rohrbündelreaktor mit 5 parallelen Rohren (Länge jeweils 12 m, Durchmesser jeweils 40 mm) durchgeführt. In jedem der Rohre waren 5,7 kg Katalysatorschüttung enthalten. Als Zulauf in den Epoxidationsreaktor wurde in allen 3 Versuchen das folgende Gemisch verwendet:

| **Stoff** | **Flussrate / (kg/h)** | **%** |
|---|---|---|
| Propen | 11,3 | 11 |
| (chemical grade) | | |
| Methanol | 74,26 | 72 |
| (98 %ig) | | |
| H₂O₂ | 17 | 17 |
| (40 %ig) | | |

Der Festbettreaktor wurde von unten nach oben durchströmt. Die Kühlung des Reaktors erfolgte über den Mantelraum mit Kühlwasser.

### Beispiel 1: Verwendung eines einzigen Katalysators (Vergleichsbeispiel)

Der Festbettrohrreaktor wurde mit Katalysator-Formkörpern gefüllt. Als katalytisch aktive Komponente enthielten die Formkörper ein zeolithisches Material (Titansilikalit des Strukturtyps TS-1). Das zeolithische Material hatte einen Titangehalt, berechnet als TiO₂, von 2,0 Gew.-%. Das molare Verhältnis von SiO₂ zu TiO₂ betrug 59.

Die Katalysator-Formkörper, die diesen Zeolithen enthielten, wiesen einen Titangehalt, berechnet als TiO₂, von 1,2 Gew.-%, und einen SiO₂-Gehalt von 98,8 Gew.-% auf. Die Formkörper waren Extrudate mit einem Durchmesser von 1,5 mm und einem Schüttgewicht von 470 g/l. Die Seitendruckfestigkeit der Formkörper betrug 9,1 N, bestimmt mit einem Messgerät der Firma mit Fa. Zwick, Modell BZ2.5/TS1S, wobei die Vorkraft 0,5 N, die Vorkraftgeschwindigkeit 10 mm/min und die Prüfgeschwindigkeit 1,6 mm/min betrugen. Das Gesamtporenvolumen der Formkörper betrug 0,73 ml/g, bestimmt über DIN 66133, die spezifische Oberfläche 397 m²/g, bestimmt über DIN 66131.

Bei der Epoxidierung bei einem Druck von 20 bar, einer Peaktemperatur von ungefähr 56 °C und einer Kühlwassertemperatur von 45 °C wurde eine Propylenoxid-Ausbeute, bezogen auf eingesetztes H₂O₂, von 91 % erreicht.

### Beispiel 2: Verwendung von zwei verschiedenen Katalysatoren

Der Festbettrohrreaktor wurde mit Katalysator-Formkörpern gefüllt. Als katalytisch aktive Komponente enthielten die Formkörper ein zeolithisches Material (Titansilikalit des Strukturtyps TS-1). Dabei wurden zwei unterschiedliche Formkörpertypen verwendet.

Als untere Lage im Reaktor wurden 4,0 kg der folgenden Formkörper eingefüllt:

Das in den Formkörpern enthaltene zeolithische Material hatte einen Titangehalt, berechnet als TiO₂, von 2,3 Gew.-%. Das molare Verhältnis von SiO₂ zu TiO₂ betrug 52. Das Porenvolumen des zeolithischen Materials, bestimmt über DIN 66134, betrug 0,497 ml/g, die spezifische Oberfläche, bestimmt über DIN 66131, 508 m²/g.

Die Katalysator-Formkörper, die diesen Zeolithen enthielten, wiesen einen Titangehalt, berechnet als TiO₂, von 2,0 Gew.-%, und einen SiO₂-Gehalt von 98,0 Gew.-% auf. Die Formkörper waren Extrudate mit einem Durchmesser von 1,5 mm und einem Schüttgewicht von 498 g/l. Die Seitendruckfestigkeit der Formkörper betrug 27 N, bestimmt mit einem Messgerät der Firma mit Fa. Zwick, Modell BZ2.5/TS1S, wobei die Vorkraft 0,5 N, die Vorkraftgeschwindigkeit 10 mm/min und die Prüfgeschwindigkeit 1,6 mm/min betrugen. Das Gesamtporenvolumen der Formkörper betrug 0,78 ml/g, bestimmt über DIN 66133, die spezifische Oberfläche 190 m²/g, bestimmt über DIN 66131. Nach der eigentlichen Herstellung der Formkörper wurden diese vor dem Einsatz im Verfahren für 12 h bei 130 °C mit Wasser in Kontakt gebracht.

Als obere Lage im Reaktor wurden 1,7 kg der Formkörper gemäß Beispiel 1 eingefüllt.

Die beiden Katalysatorlagen grenzten direkt aneinander und waren durch keine physikalische Trenneinrichtung räumlich voneinander getrennt.

Bei der Epoxidierung bei einem Druck von 20 bar, einer Peaktemperatur von ungefähr 56 °C und einer Kühlwassertemperatur von 45 °C wurde eine Propylenoxid-Ausbeute, bezogen auf eingesetztes H₂O₂, von 92,2 % erreicht.

### Beispiel 3: Verwendung von zwei verschiedenen Katalysatoren

Der Festbettrohrreaktor wurde mit Katalysator-Formkörpern gefüllt. Als katalytisch aktive Komponente enthielten die Formkörper ein zeolithisches Material (Titansilikalit des Strukturtyps TS-1). Dabei wurden zwei unterschiedliche Formkörpertypen verwendet.

Als untere Lage im Reaktor wurden 5,0 kg der folgenden Formkörper eingefüllt:

Das in den Formkörpern enthaltene zeolithische Material hatte einen Titangehalt, berechnet als TiO₂, von 2,3 Gew.-%. Das molare Verhältnis von SiO₂ zu TiO₂ betrug 52. Das Porenvolumen des zeolithischen Materials, bestimmt über DIN 66134, betrug 0,497 ml/g, die spezifische Oberfläche, bestimmt über DIN 66131, 508 m²/g.

Die Katalysator-Formkörper, die diesen Zeolithen enthielten, wiesen einen Titangehalt, berechnet als TiO₂, von 2,0 Gew.-%, und einen SiO₂-Gehal von 98,0 Gew.-% auf. Die Formkörper waren Extrudate mit einem Durchmesser von 1,5 mm und einem Schüttgewicht von 498 g/l. Die Seitendruckfestigkeit der Formkörper betrug 17 N, bestimmt mit einem Messgerät der Firma mit Fa. Zwick, Modell BZ2.5/TS1S, wobei die Vorkraft 0,5 N, die Vorkraftgeschwindigkeit 10 mm/min und die Prüfgeschwindigkeit 1,6 mm/min betrugen. Das Gesamtporenvolumen der Formkörper betrug 0,88 ml/g, bestimmt über DIN 66133, die spezifische Oberfläche 313 m²/g, bestimmt über DIN 66131. Nach der eigentlichen Herstellung der Formkörper wurden diese vor dem Einsatz im Verfahren für 36 h bei 140 °C mit Wasser in Kontakt gebracht.

Als obere Lage im Reaktor wurden 0,7 kg der Formkörper gemäß Beispiel 1 eingefüllt.

Die beiden Katalysatorlagen grenzten direkt aneinander und waren durch keine physikalische Trenneinrichtung räumlich voneinander getrennt.

Bei der Epoxidierung bei einem Druck von 20 bar, einer Peaktemperatur von ungefähr 55 °C und einer Kühlwassertemperatur von 43 °C wurde eine Propylenoxid-Ausbeute, bezogen auf eingesetztes H₂O₂, von 93,4 % erreicht.

## Patentansprüche

1. Verfahren zur katalysierten Umsetzung einer organischen Verbindung mit einem Hydroperoxid in mindestens einem Reaktor unter Verwendung von mindestens zwei voneinander verschiedenen Titanzeolith-Katalysatoren, **dadurch gekennzeichnet, dass** mindestens zwei der voneinander verschiedenen Zeolith-Katalysatoren räumlich voneinander getrennt eingesetzt werden, und wobei sich die Titanzeolith-Katalysatoren im Titangehalt oder in der Zeolithstruktur oder sowohl im Titangehalt als auch der Zeolithstruktur unterscheiden.

2. Verfahren nach Anspruch 1, **dadurch gekennzeichnet, dass** mindestens ein Katalysator ein Titanzeolithkatalysator vom Kristallstrukturtyp MWW ist und mindestens ein Katalysator ein Titanzeolithkatalysator vom Kristallsttukturlyp MFI ist.

3. Verfahren nach Anspruch 1 oder 2, **dadurch gekennzeichnet, dass** die Umsetzung der organischen Verbindung in einem einzigen Reaktor durchgeführt wird.

4. Verfahren nach Anspruch 3, wobei der Reaktor ein kontinuierlich betriebener isothermer Festbettrohrreaktor ist.

5. Verfahren nach Anspruch 1 oder 2, **dadurch gekennzeichnet, dass** die Umsetzung in mindestens zwei seriell geschalteten Reaktoren durchgeführt wird, wobei in mindestens einem ersten Reaktor mindestens ein Titanzeolith-Katalysator und in mindestens einem zweiten Reaktor mindestens ein weiterer Titanzeolith-Katalysator eingesetzt wird, der sich von mindestens einem der in dem ersten Reaktor eingesetzten Titanzeolith-Katalysatoren unterscheidet.

6. Verfahren nach Anspruch 5, **dadurch gekennzeichnet, dass** mindestens einer der eingesetzten Reaktoren in Festbettfahrweise betrieben wird.

7. Verfahren nach Anspruch 6, wobei die Umsetzung in zwei seriell gekoppelten Reaktoren durchgeführt wird und die erste Umsetzung in einem isothermen Festbettrohrreaktor und die zweite Umsetzung in einem adiabatischen Festbettrohrreaktor stattfindet.

8. Verfahren nach einem der Ansprüche 1 bis 7, **dadurch gekennzeichnet, dass** als organische Verbindung ein Alken und als Hydroperoxid Wasserstoffperoxid eingesetzt wird.

9. Verfahren nach Anspruch 8, **dadurch gekennzeichnet, dass** als organische Verbindung Propen eingesetzt wird.

10. Kontinuierlich betriebener isothermer Festtbettrohrreaktor zur Umsetzung einer organischen Verbindung mit einem Hydroperoxid, enthaltend mindestens zwei räumlich voneinander getrennte, voneinander verschiedene Titanzeolith-Katalysatoren, wobei sich die Titanzeolith-Katalysatoren im Titangehalt oder in der Zeolithstruktur oder sowohl im Titangehalt als auch der Zeolithstruktur unterscheiden.

11. Festbettreaktor nach Anspruch 10 zur Umsetzung von Propen mit Wasserstoffperoxid.

12. Seriell gekoppelter Reaktorverbund aus zwei seriell gekoppelten Reaktoren zur Umsetzung einer organischen Verbindung mit einem Hydroperoxid, wobei der erste Reaktor ein isothermer Festbettrohrreaktor und der zweite Reaktor ein adiabatischer Festbettrohrreaktor ist, wobei die zwei Reaktoren jeweils voneinander verschiedene Titanzeolith-Katalysatoren enthalten, und wobei sich die Titanzeolith-Katalysatoren im Titangehalt oder in der Zeolithstruktur oder sowohl im Titangehalt als auch der Zeolithstruktur unterscheiden.

13. Reaktorverbund nach Anspruch 12 zur Umsetzung von Propen mit Wasserstoffperoxid.

## Claims

1. A process for the catalyzed reaction of an organic compound with a hydroperoxide in at least one reactor using at least two different titanium zeolite catalysts, wherein at least two of the different zeolite catalysts are used physically separately from one another and the titanium zeolite catalysts differ in the titanium content or in the zeolite structure or both in the titanium content and in the zeolite structure.

2. The process according to claim 1, wherein at least one catalyst is a titanium zeolite catalyst of the MWW crystal structure type and at least one catalyst is a titanium zeolite catalyst of the MFI crystal structure type.

3. The process according to claim 1 or 2, wherein the reaction of the organic compound is carried out in a single reactor.

4. The process according to claim 3, wherein the reactor is a continuously operated isothermal fixed-bed tube reactor.

5. The process according to claim 1 or 2, wherein the reaction is carried out in at least two reactors connected in series, where at least one titanium zeolite catalyst is used in at least one first reactor and at least one further titanium zeolite catalyst which differs from at least one of the titanium zeolite catalysts used in the first reactor is used in at least one second reactor.

6. The process according to claim 5, wherein at least one of the reactors used is operated in the fixed-bed mode.

7. The process according to claim 6, wherein the reaction is carried out in two reactors connected in series and the first reaction takes place in an isothermal fixed-bed tube reactor and the second reaction takes place in an adiabatic fixed-bed tube reactor.

8. The process according to any of claims 1 to 7, wherein an alkene is used as organic compound and hydrogen peroxide is used as hydroperoxide.

9. The process according to claim 8, wherein propene is used as organic compound.

10. A continuously operated isothermal fixed-bed tube reactor for reacting an organic compound with a hydroperoxide, which comprises at least two physically separate titanium zeolite catalysts which are different from one another, wherein the titanium zeolite catalysts differ in the titanium content or in the zeolite structure or both in the titanium content and in the zeolite structure.

11. The fixed-bed reactor according to claim 10 for reacting propene with hydrogen peroxide.

12. A reactor assembly comprising two reactors connected in series for reacting an organic compound with a hydroperoxide, wherein the first reactor is an isothermal fixed-bed tube reactor and the second reactor is an adiabatic fixed-bed tube reactor, the two reactors comprise different titanium zeolite catalysts and the titanium zeolite catalysts differ in the titanium content or in the zeolite structure or both in the titanium content and in the zeolite structure.

13. The reactor assembly according to claim 22 for reacting propene with hydrogen peroxide.

## Revendications

1. Procédé de réaction catalysée d'un composé organique avec un hydroperoxyde dans au moins un réacteur en utilisant au moins deux catalyseurs à base de zéolithe au titane différents l'un de l'autre, **caractérisé en ce qu'**au moins deux des catalyseurs à base de zéolithe au titane différents l'un de l'autre sont utilisés séparément l'un de l'autre dans l'espace, les catalyseurs à base de zéolithe au titane différant au niveau de la teneur en titane ou de la structure de la zéolithe ou aussi bien au niveau de la teneur en titane que de la structure de la zéolithe.

2. Procédé selon à revendication 1, **caractérisé en ce qu'**au moins un catalyseur est un catalyseur à base de zéolithe au titane de type de structure cristalline MWW et au moins un catalyseur est un catalyseur à base de zéolithe au titane de type de structure cristalline MFI.

3. Procédé selon la revendication 1 ou 2, **caractérisé en ce que** la réaction du composé organique est réalisée dans un réacteur individuel.

4. Procédé selon la revendication 3, **caractérisé en ce que** le réacteur est un réacteur tubulaire à lit fixe isotherme exploité en continu.

5. Procédé selon la revendication 1 ou 2, **caractérisé en ce que** la réaction est réalisée dans au moins deux réacteurs connectés en série, au moins un catalyseur à base de zéolithe au titane étant utilisé dans au moins un premier réacteur et au moins un autre catalyseur à base de zéolithe au titane, qui diffère d'au moins un des catalyseurs à base de zéolithe au titane utilisés dans le premier réacteur, étant utilisé dans au moins un deuxième réacteur.

6. Procédé selon la revendication 5, **caractérisé en ce qu'**au moins un des réacteurs utilisés est exploité en mode à lit fixe.

7. Procédé selon la revendication 6, **caractérisé en ce que** la réaction est réalisée dans deux réacteurs couplés en série et la première réaction a lieu dans un réacteur tubulaire à lit fixe isotherme et la deuxième réaction dans un réacteur tubulaire à lit fixe adiabatique.

8. Procédé selon l'une quelconque des revendications 1 à 7, **caractérisé en ce qu'**un alcène est utilisé en tant que composé organique ét du peroxyde d'hydrogène en tant qu'hydroperoxyde.

9. Procédé selon la revendication 8, **caractérisé en ce que** du propène est utilisé en tant que composé organique.

10. Réacteur tubulaire à lit fixe isotherme exploité en continu pour la réaction d'un composé organique avec un hydroperoxyde, contenant au moins deux catalyseurs à base de zéolithe au titane différents l'un de l'autre, séparés l'un de l'autre dans l'espace, les catalyseurs à base de zéolithe au titane différant au niveau de la teneur en titane ou de la structure de la zéolithe ou aussi bien au niveau de la teneur en titane que de la structure de la zéolithe.

11. Réacteur à lit fixe selon la revendication 10 pour la réaction de propène avec du peroxyde d'hydrogène.

12. Interconnexion de réacteurs couplés en série constituée de deux réacteurs couplés en série pour la réaction d'un composé organique avec un hydroperoxyde, le premier réacteur étant un réacteur tubulaire à lit fixe isotherme et le deuxième réacteur étant un réacteur tubulaire à lit fixe adiabatique, les deux réacteurs contenant chacun des catalyseurs à base de zéolithe au titane différents les uns des autres, et les catalyseurs à base de zéolithe au titane différant au niveau de la teneur en titane ou de la structure de la zéolithe ou aussi bien au niveau de la teneur en titane que de la structure de la zéolithe.

13. Interconnexion de réacteurs selon la revendication 12 pour la réaction de propène avec du peroxyde d'hydrogène.
